# DEMANDE DE BREVET EUROPEEN

(11) **EP 0 526 910 A2**
(43) Date de publication de la demande: **10.02.1993**
(21) Numéro de dépôt: 92117254.0
(22) Date de dépôt: 11.07.1990
(51) Int. Cl.: C07H 15/04

(54) **Nouveau procédé de préparation d'alkyl-glycosides**

(30) Priorité: 31.07.1989 FR 8910301
(62) Demande divisionnaire de: 90401991.6
(71) Demandeur: ERIDANIA BEGHIN-SAY, F-59239 Thumeries (FR)
(72) Inventeur: Defaye, Jacques, F-38330 Saint Dismier (FR); Gadelle, Andrée, F-38330 Saint Dismier (FR); Pedersen, Christian, DK-2830 Virum (DK)
(74) Mandataire: David, Daniel

(57) **Abrégé**

Procédé de préparation d'alkyl-glycosides à partir d'un saccharide choisi parmi les monosaccharides, les di- et les oligo-saccharides réducteurs caractérisé par le fait que l'on fait réagir ledit saccharide avec, respectivement un alcool dans le solvant et réactif poly- (fluorure d'hydrogène) - pyridinium.

## Description

La présente invention se rapporte à un nouveau procédé de préparation d'alkyl-glycosides. Plus particulièrement, l'invention concerne un procédé amélioré de préparation d'alkyl-glycosides, notamment à longue chaîne alkyle ou chaîne grasse, par action d'un alcool sur un saccharide choisi parmi les monosaccharides, les di- et oligo-saccharides réducteurs dans le solvant et réactif poly(fluorure d'hydrogène)- pyridinium.

Les alkyl-glycosides à chaîne grasse sont connus pour leurs propriétés d'agents tensio-actifs, et en particulier pour leur utilisation comme surfactants ou détergents. Une caractéristique essentielle de ces familles de dérivés glucidiques est de réunir, simultanément sur la même molécule, des groupes fonctionnels hydrophiles et hydrophobes, ce qui leur permet de former des couches monomoléculaires aux interfaces de solvants non miscibles, et des micelles lorsque leur concentration en solution excède une valeur limitante connue sous le nom de concentration micellaire critique (CMC). Ce comportement physico-chimique est à l'origine des applications extrêmement importantes, aussi bien que diverses, des surfactants comme agents de mouillage, de dispersion, comme agents émulsifiants, moussants et de façon plus générale comme agents de solubilisation. Les surfactants sont également connus pour leurs propriétés thermotropiques et lyotropiques de cristaux liquides.

Les alkyl-glycosides à chaîne grasse constituent une classe de détergents tout particulièrement intéressante car, étant non ioniques, leur CMC, et donc leurs propriétés, ne sont pas dépendantes de la présence d'un contre-ion. La valeur généralement élevée de leur CMC permet par ailleurs de les éliminer commodément et rapidement par dialyse. Ces caractéristiques ont notamment conduit à privilégier leur utilisation en biochimie, pour la solubilisation, l'extraction et la reconstitution de protéines membranaires, et ce d'autant plus que ces dérivés amphiphiles n'ont généralement pas ou peu d'effets dénaturants sur ces structures biologiques complexes et labiles.

Les alkyl-glycosides à chaîne grasse sont le plus généralement préparés par des procédés de trans-acétalation en présence d'un catalyseur acide, à partir d'un glycoside d'alcool inférieur, ou encore d'un mélange de l'ose avec un alcool inférieur et un alcool gras, comme cela a été décrit dans les brevets DE-A-1905523 et DE-A-1943689. Un procédé apparenté à la réaction de Koenigs-Knorr et qui fait intervenir l'action d'un alcool à longue chaîne sur un halogénure de glycosyle peracétylé, en présence de sels d'argent, a également été préconisé par Koeltzow et al. dans J. Am. Oil Chem. Soc. 61 (1984) 1651 et conduit aux anomères β du cellobiose, du maltose et du maltotriose.

Le WO-A-8600906 de la demanderesse décrit un procédé de synthèse d'alkyl-glycosides d'alcools gras par réaction d'un alcool gras avec un aldose, un aldoside ou un polyaldoside dans un solvant et réactif constitué par un mélange dioxanne-fluorure d'hydrogène ou dioxyde de soufre-fluorure d'hydrogène mais outre le fait que les rendements obtenus sont relativement faibles, de l'ordre de 30 %, ce procédé n'est pas utilisable pour la synthèse d'alkyloligosaccharides car les conditions qui y sont employées conduisent à la fluorolyse des liaisons interosidiques oxygénées. Des alkylglycosides d'alcools inférieurs sont également synthétisés, avec un bon rendement, selon le WO-A-8600906 précité par réaction d'un alcool avec un aldose, un aldoside ou un polyaldoside dans un solvant et réactif constitué par du fluorure d'hydrogène mais la quantité d'alcool utilisé est élevée, le rapport molaire alcool sur équivalent monosaccharidique étant avantageusement de l'ordre de 20 et les alkyl-oligosaccharides inférieurs ne peuvent pas non plus être obtenus pour la raison indiquée précédemment.

Un objet de la présente invention est de préparer en une seule étape des alkyl-oligosaccharides.

Un autre objet de l'invention est d'obtenir des alkyl-glycosides d'alcools à chaîne grasse en une étape, avec un bon rendement, en partant aussi bien d'un monosaccharide que d'un di- ou d'un oligo-saccharide.

Un objet de l'invention est aussi de préparer des alkyl-glycosides d'alcools inférieurs en faisant réagir un saccharide avec un faible excès d'alcool par rapport à la stoechiométrie.

D'autres objets et avantages de l'invention apparaîtront à la lecture de la description ci-après.

La présente invention répond aux objets précités et fournit un procédé de préparation en une étape d'alkyl-glycosides, à partir d'un saccharide choisi parmi les monosaccharides, les di- et oligosaccharides réducteurs, caractérisé par le fait que l'on fait réagir ledit saccharide avec un alcool dans le solvant et réactif poly- (fluorure d'hydrogène) - pyridinium. Le produit résultant, alkyl-glycoside, qui se forme rapidement avec un excellent rendement, est ensuite extrait. Le poly- (fluorure d'hydrogène) - pyridinium, encore connu sous le nom de réactif fluorure d'hydrogène-pyridine, est un produit commercial, qui peut encore être préparé selon le procédé décrit par Olah et al. dans J. Org. Chem. 44 (1979) 3872.

Le procédé de l'invention permet l'obtention en une étape d'alkyl-glycosides à chaîne grasse ainsi que celle d'alkyl-glycosides d'alcools inférieurs, en partant aussi bien d'un monosaccharide que d'un di- ou d'un oligo-saccharide réducteur.

Parmi les monosaccharides, on peut utiliser aussi bien les hexoses et les pentoses, et en particulier le D-glucose du fait de son accessibilité, ou encore un amino-désoxy-hexose tel que le 2-acétamido-2-désoxy-D-glucose. En ce qui concerne les di- et oligo-saccharides, et pour les mêmes raisons d'accessibilité, des disaccharides tels que le cellobiose, le lactose et le maltose sont les plus susceptibles d'utilisation, mais des oligo-saccharides de degré de polymérisation supérieur comportant jusqu'à dix motifs monosaccharidiques, et même au-delà, peuvent également être utilisés si les propriétés de détergence attendues du produit final le justifient.

Pour la préparation des alkyl-glycosides, il est préférable d'utiliser un excès qui peut aller jusqu'à 2 à 3 moles du réactant alcool par mole du réactant saccharide.

La quantité du solvant et réactif poly-(fluorure d'hydrogène)-pyridinium n'est pas critique, elle doit simplement être suffisante pour permettre de travailler en phase homogène. Si l'on a constaté qu'il n'y avait aucun avantage à augmenter la quantité du solvant et réactif poly-(fluorure d'hydrogène) - pyridinium au- delà de la quantité nécessaire à l'homogénéité du milieu réactionnel, il faut toutefois noter qu'aucun inconvénient, hormis l'élévation du prix de revient, ne résulte d'une telle augmentation.

Le procédé de l'invention permet d'obtenir des alkylglycosides à chaîne grasse ainsi qu'à chaîne courte partant des alcools correspondants. Pour l'obtention d'alkyl-glycosides à longue chaîne, le réactant alcool sera un alcool à chaîne grasse comportant de façon optimale de 5 à 20 atomes de carbone. Là encore, on devra tenir compte dans le choix de l'alcool, de l'équilibre souhaité entre les propriétés d'hydrophilie et de lipophilie du produit final.

Pour l'obtention d'alkyl-glycosides à chaîne grasse selon l'invention, l'alcool gras correspondant est utilisé de préférence avec un excès de 2 à 3 moles par mole du réactant saccharide. Les rendements sont supérieurs à ceux des procédés de l'art antérieur. La présente invention permet en outre l'obtention d'alkyl-oligosaccharides qui ne pouvaient être préparés en une étape par les procédés de l'art antérieur.

Le procédé de l'invention s'avère également avantageux pour l'obtention d'alkyl-glycosides d'alcools inférieurs comportant notamment un marquage isotopique sur le résidu aglyconique du fait de la faible quantité de réactant aglyconique mise en jeu.

Les Exemples suivants, qui donnent des modes opératoires types pour préparer certains des produits revendiqués, illustrent l'invention sans la limiter.

### Exemple 1 : Préparation du 1-hexyl-α,β-D-glucopyranoside.

Dans un récipient en Téflon, le D-glucose (1 g, 5,5 mmol) est additionné de 1-hexanol (1,4 ml, 12 mmol) et du réactif commercial poly -(fluorure d'hydrogène)- pyridinium (10 ml). Après une agitation magnétique d'une heure à température ambiante, on ajoute de l'éther diéthylique (100 ml) et du carbonate de calcium (20 g). Les sels minéraux insolubles sont alors séparés par filtration et le filtrat est concentré sous pression réduite. L'huile résiduelle est reprise par l'éther diéthylique et la solution éthérée est lavée par l'eau et séchée sur sulfate de sodium. Par concentration, on obtient le 1-hexyl-D-glucopyranoside (830 mg, 50 %) sous la forme d'une huile qui contient les anomères α et β dans un rapport relatif 7:3 tel qu'estimé par l'intégration des signaux en r.m.n. du ¹³C : [α]D + 103,2° (c 1,6, DMSO).

R.m.n. ¹³C (50,323 MHz,D₂0, δ p.p.m. avec comme référence le CH₃ hexyle à δ 14 p.p.m.): anomère β; 102,8 (C-1); 76,7, 76,3, 72,3, 70,5 (C-2_C-5); 61,6 (C-6); 68,5 (OCH₂) 31,3, 29,2, 25,3, 22,4 (4 CH₂); 14 (CH₃): anomère α : 98,7 (C-1); 74,2 (2), 72,2, 70,4 (C-2 C-5); 61,5 (C-6); 68,5 (OCH₂); 31,3: 29,2, 25,5 : 22,4 (4 CH2); 14 (CH₃).

### Exemple 2 : Préparation du 1-hexyl-α,β-cellobioside

Dans un récipient en Téflon, le cellobiose (1g. 2.9 mmol) est additionné d'hexanol (1,4 ml, 7,7 mmol) et du réactif commercial poly- (fluorure d'hydrogène)- pyridinium (5 ml). Après une agitation magnétique d'une heure à température ambiante, on ajoute de l'éther diéthylique (100 ml) et du carbonate de calcium (∼5 g). Le mélange est maintenu agité pendant encore 1 h, puis il est filtré et les sels sont lavés par le méthanol (50 ml). Les filtrats réunis sont concentrés sous pression réduite et la pyridine éventuellement résiduelle est entrainée par co-évaporation avec de l'eau. Le produit brut de la réaction, obtenu sous la forme d'une huile (1,23 g) à cette étape, contient le 1-hexyl cellobioside sous la forme d'un mélange anomérique α/β, 1:1 (¹³C r.m.n.) et un peu de cellobiose non réagit. Le 1-hexyl cellobioside peut être obtenu pur (620 mg, 50 %) par passage sur une colonne de résine Dowex 1 (OH⁻) équilibrée dans le méthanol selon la technique décrite dans Biochemistry, 19 (1980) 4108.

R.m.n. ¹³C (50,323 MHz, ²H₂O, δ p.p.m. en référence à C-6 α à 60,1) : 102,9 (C'-1) ; 98,3 (C-1α) ; 102,5 (C-1β) ; 79,4 (C-4) ; 68,9 (-OCH₂-) ; 61,3 (C'-6) ; 60,1 (C-6 α) ; 60,8 (C-6 β).

### Exemple 3 : Préparation du méthyl-α,β-cellobioside

Dans un récipient en Téflon, le cellobiose (1 g, 2,9 mmol) est additionné de méthanol (0,22 ml, 7 mmol) et du réactif commercial poly-(fluorure d'hydrogène) - pyridinium (5 ml). Après une agitation magnétique d'une heure à température ambiante, on ajoute de l'éther diéthylique (100 ml) et du carbonate de calcium (∼5 g). Le mélange est maintenu agité pendant encore 1 h, puis il est additionné de méthanol (20 ml) et filtré, et les sels sont lavés par le méthanol. Les filtrats réunis sont concentrés sous pression réduite et le résidu de pyridine éventuel est entraîné par co-évaporation avec de l'eau. L'huile obtenue (1 g, 90 %) contient le mélange anomérique de méthyl-cellobioside α/β, 2:3 (r.m.n. ¹³C). R.m.n. ¹³C (50,323 MHz, ²H₂O, δ p.p.m. en référence à C-6 α à 60,80 p.p.m.) : 103,9 (C-1 β ; 103,3 (C'-1) ; 99,8 (C-1 α) ; 61,30 (C'-6) ; 60,80 (C-6 α, β) ; 57,6 (-OCH₃ β) ; 55,7 (-OCH₃ α).

## Revendications

1. Procédé de préparation d'alkyl-glycosides à partir d'un saccharide choisi parmi les monosaccharides, les di- et les oligo-saccharides réducteurs, caractérisé par le fait que l'on fait réagir ledit saccharide avec un alcool dans le solvant et réactif poly- (fluorure d'hydrogène) - pyridinium.

2. Procédé selon la revendication 1 caractérisé par le fait que ledit saccharide est un monosaccharide choisi parmi les hexoses et les pentoses.

3. Procédé selon la revendication 2 caractérisé par le fait que ledit monosaccharide est le D-glucose.

4. Procédé selon la revendication 2 caractérisé par le fait que ledit monosaccharide est un amino-désoxy-hexose.

5. Procédé selon la revendication 1 caractérisé par le fait que ledit saccharide est un disaccharide choisi parmi le cellobiose, le lactose et le maltose.

6. Procédé de préparation d'alkyl-glycosides selon la revendication 1 caractérisé par le fait que le rapport molaire entre ledit alcool et ledit saccharide est compris entre environ 2 et 3.

7. Procédé de préparation d'alkyl-glycosides à chaîne grasse selon la revendication 1 caractérisé par le fait que le nombre d'atomes de carbone dudit alcool est compris entre 5 et 20.
